# EUROPÄISCHE PATENTANMELDUNG

(11) **EP 3 144 035 A1**
(43) Veröffentlichungstag der Anmeldung: **22.03.2017**
(21) Anmeldenummer: 16189104.9
(22) Anmeldetag: 16.09.2016
(51) Int. Cl.: A61Q 17/04, A61K 8/34, A61K 8/81, A61K 8/37, A61K 8/49, A61K 31/12

(54) **GLYCERIN-HALTIGES KOSMETIKUM MIT VINYLPYRRODIDON/TRIACONTEN-COPOLYMER**

(30) Priorität: 21.09.2015 DE 102015218065
(71) Anmelder: Beiersdorf AG, 20253 Hamburg (DE)
(72) Erfinder: Weinert, Katrin, 22763 Hamburg (DE); Borchers, Kathrin, 21614 Buxtehude (DE); Bleckmann, Andreas, 22926 Ahrensburg (DE); Lerg, Heike, 22303 Hamburg (DE)

(57) **Zusammenfassung**

Kosmetische Zubereitung enthaltend
a) Glycerin und
b) Vinylpyrrolidon/Triaconten-Copolymer.

## Beschreibung

Die vorliegende Erfindung betrifft eine kosmetische Zubereitung enthaltend Glycerin und Vinylpyrrolidon/Triaconten-Copolymer.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie 4-(tert.-Butyl)-4'-methoxydibenzoylmethan, (2-[-4-(Diethylamino)-2-hydroxybenzoyl] Benzoesäurehexylester 2,4-Bis-{[4-(2-ethyl-hexyloxy)-2-hydroxy]-phenyl}-6-(4-methoxyphenyl)-1,3,5-triazin (INCl Bis-Ethylhexyloxyphenol Methoxyphenyl Triazine) hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche UV-A- und/oder Breitbandfilter wie in 4-(tert.-Butyl)-4'-methoxy-dibenzoylmethan, (2-[-4-(Diethylamino)-2-hydroxybenzoyl] Benzoesäurehexylester (INCl: Diethylamino Hydroxybenzoyl Hexyl Benzoate) und/oder Bis-Ethylhexyloxyphenol Methoxyphenyl Triazine) zu entwickeln, welche sich leichter aus den mit der Zubereitung kontaminierten Textilen herauswaschen lassen.

Eine besondere Form kosmetischer Zubereitungen (z.B. Sonnenschutzmittel) stellen die sprühbare Zubereitungen dar, die mit Hilfe eines Sprühapplikators mit Sprühkopf entweder mit Hilfe einer mechanischen Förderpumpe oder mittels eines Treibgases aus einem Vorratsbehältnis auf die Haut appliziert werden. An derartige Zubereitungen werden eine Reihe von Anforderungen gestellt, die in ihrer Gesamtheit oft nur schwer zu vereinbaren sind.

So sollen sich die Zubereitungen angenehm auf der Haut anfühlen, gut zu versprühen sein (d.h. leicht aus dem Vorratsbehälter zu entnehmen sein und sich dabei gleichmäßig, großflächig auf der Haut verteilen) und dabei nicht so dünnflüssig sein, dass sie nach dem Auftragen auf die Haut sofort herunterfließen, sondern ein gewisses "Haftungsvermögen" auf der Haut aufzeigen, um anschließend wiederum einfach und gleichmäßig auf der Haut zu verteilen sein.

Dabei bereitet es insbesondere Schwierigkeiten eine Balance aus guter Sprühbarkeit hinsichtlich des Sprühbildes (d.h. eine gleichmäßige, großflächige Verteilung der Zubereitung auf der Haut in Form kleiner, gleichförmiger Tröpfchen) und der Viskosität bzw. des Fließverhaltens der Zubereitung und ihrer Verteilbarkeit nach dem Auftragen auf der Haut zu gewährleisten. Zubereitungen des Standes der Technik geraten dabei entweder so dünnflüssig, dass sie beim Pumpspray in Form eines fokussierten Strahls "Wasserpistolen gleich" aus dem Applikator austreten oder die Zubereitungen sind so zähflüssig, dass sie sich nicht mehr richtig versprühen und auf der Haut verteilen lassen.

Es war daher die Aufgabe der vorliegenden Erfindung, ein sprühbares Kosmetikum (insbesondere ein Sonnenschutzmittel) zu entwickeln, dass insbesondere für den Einsatz in Pumpsprays geeignet ist, welches eine gleichmäßige, großflächige Verteilung auf der Haut in Form kleiner, gleichförmiger Tröpfchen ermöglicht ohne so dünnflüssig zu sein, dass es nach dem Auftrag sofort von der Haut heruntertropft. Dabei sollte die Zubereitung sensorisch angenehm und leicht auf der Haut zu verteilen sein.

Da dem Fachmann natürlich bekannt ist, dass auch die Ausgestaltung des Sprühkopfes des Sprühapplikators einen Einfluss auf das Sprühbild hat, bezieht sich der Vergleich der Sprühbilder natürlich immer auf die Verwendung des gleichen Sprühkopfes. Die beschriebene Aufgabe sollte jedoch unabhängig vom Sprühkopf-Typ für alle in der Kosmetik üblichen Sprühköpfe gelöst werden.

Überraschend gelöst werden die Aufgaben durch eine kosmetische Zubereitung enthaltend
a) Glycerin und
b) Vinylpyrrolidon/Triaconten-Copolymer.

Erfindungsgemäß bevorzugt handelt es sich bei der kosmetischen Zubereitung um ein Sonnenschutzmittel, enthaltend einen oder mehrere UV-Filter.

Es war insbesondere überraschend, dass die Auswaschbarkeit der durch die UV-Filter hervorgerufenen Textilverfleckungen verbessert (erleichtert) wird, obwohl Vinylpyrrolidon/Triaconten-Copolymer eigentlich zur Erhöhung der Wasserfestigkeit kosmetischer Zubereitungen (insbesondere Sonnenschutzmittel) auf der Haut eingesetzt wird und die erfindungsgemäßen Zubereitungen auch eine erhöhte Wasserfestigkeit der Zubereitung auf der Haut aufweisen. Dieser Unterschied von Auswaschbarkeit aus Textilen und Wasserfestigkeit auf der Haut war für den Fachmann so nicht vorhersehbar.

Zwar kennt der Fachmann die Produktinformation "Antaron WP-660, Specialty Products for Personal Care" der Firma Ashland sowie das Produkt "Sun Protective Lotion SPF 40 for Kids" der Marke "Calypso Sun" der Firma Linco Care (GNPD Datenbank "Mintel", Eintragungsnummer 2119401), doch konnten diese Publikationen nicht den Weg zur vorliegenden Erfindung weisen, da in keiner der beiden Publikationen eine Kombination mit Glycerin offenbart wird.

Darüber hinaus weist das Produkt "Sun Protective Lotion SPF 40 for Kids der Marke Calypso Sun" der Firma Linco Care (GNPD Datenbank "Mintel", Eintragungsnummer 2119401) eine Viskosität von 18200 mPa*s auf (Messmethode, siehe unten) und ist damit nicht mehr sprühbar.

Nicht zuletzt führt dieses Produkt nach dem Waschen zu einer relativ starken Textilverfleckung (siehe Vergleichsversuch) auch wenn es die Produktauslobung "non staining formulation" trägt. Während es bei diesem Produkt des Standes der Technik nach dem Waschen eines mit der Zubereitung kontaminierten Textils (0,25g auf Baumwoll-Stoffmonitor; gemäß beschriebener Verfleckungsuntersuchungsmethode) zu einem Anstieg der Verfärbung durch die Zubereitung kommt, zeichnen sich die erfindungsgemäßen Produkte dadurch aus, dass die Verfärbung des Textils nach dem Waschen signifikant abnimmt.

| Muster | t₁: Verfleckung vor dem Waschen (db) | t₂: Verfleckung nach dem Waschen (db) | Ergebnis |
|---|---|---|---|
| Referenz Calypso Sun | 8,6 | 9,7 | t₁ < t₂ |
| Beispiel 3 | 7,5 | 5,2 | t₁ > t₂ |

Hinsichtlich der Beispielrezeptur in der Produktinformation "Antaron WP-660, Specialty Products for Personal Care" ist zu bemerken, dass diese Zubereitung nicht als Spray vorgesehen ist und keinen der für die Textilverfleckung besonders problematischen UV-Filter enthält.

Es ist erfindungsgemäß vorteilhaft, wenn die erfindungsgemäße Zubereitung einen oder mehrere UV-Filter gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxy-siloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCl: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCl: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCl: Ethylhexyl Triazone); 2,4,6-Tribiphenyl-4-yl-1,3,5-triazin; Merocyanine; Titandioxid; Zinkoxid, enthält.

Dabei ist es allerdings erfindungsgemäß bevorzugt, wenn die erfindungsgemäße Zubereitung fei ist von 3-(4-Methylbenzyliden)-campher 2-Hydroxy-4-methoxybenzophenon (Oxybenzon) und 4-Methoxyzimtsäure(2-ethylhexyl)ester.

Erfindungsgemäß vorteilhafte Ausführungsformen der vorliegenden Erfindung sind dadurch gekennzeichnet, dass die Zubereitung 2-Phenylbenzimidazol-5-sulfonsäure und/oder deren Salze enthält. Dabei sind die Alkalisalze (Natrium, Kalium) erfindungsgemäß bevorzugt.

In einem solchen Falle ist es erfindungsgemäß von Vorteil, wenn die 2-Phenylbenzimidazol-5-sulfonsäure bzw. deren Salze in einer Konzentration von 0,1 bis 5 Gewichts-%, bezogen auf das Gesamtgewicht der Zubereitung, in dieser enthalten sind.

Es ist erfindungsgemäß von Vorteil, wenn die erfindungsgemäße Zubereitung einen oder mehrere UV-Filter gewählt aus der Gruppe der Verbindungen 4-(tert.-Butyl)-4'-methoxydibenzoylmethan, (2-[-4-(Diethylamino)-2-hydroxybenzoyl] Benzoesäurehexylester und/oder 2,4-Bis-{[4-(2-ethyl-hexyloxy)-2-hydroxy]-phenyl}-6-(4-methoxyphenyl)-1,3,5-triazin (INCl Bis-Ethylhexyloxyphenol Methoxyphenyl Triazine), enthält.

Enthält die erfindungsgemäße Zubereitung 4-(tert.-Butyl)-4'-methoxydibenzoylmethan, so ist es erfindungsgemäß vorteilhaft, wenn diese Verbindung in einer Konzentration von 0,1 bis 5 Gewichts-%, bezogen auf das Gesamtgewicht der Zubereitung, in dieser enthalten ist.

Enthält die erfindungsgemäße Zubereitung (2-[-4-(Diethylamino)-2-hydroxybenzoyl] Benzoesäurehexylester, so ist es erfindungsgemäß vorteilhaft, wenn diese Verbindung in einer Konzentration von 0,1 bis 10 Gewichts-%, bezogen auf das Gesamtgewicht der Zubereitung, in dieser enthalten ist.

Enthält die erfindungsgemäße Zubereitung Bis-{[4-(2-ethyl-hexyloxy)-2-hydroxy]-phenyl}-6-(4-methoxyphenyl)-1,3,5-triazin (INCl Bis-Ethylhexyloxyphenol Methoxyphenyl Triazine), so ist es erfindungsgemäß vorteilhaft, wenn diese Verbindung in einer Konzentration von 0,1 bis 10 Gewichts-%, bezogen auf das Gesamtgewicht der Zubereitung, in dieser enthalten ist.

Erfindungsgemäß vorteilhafte Ausführungsformen der vorliegenden Erfindung sind ferner dadurch gekennzeichnet, dass die Zubereitung Ethylhexylsalicylat und/oder Homomenthylsalicylat enthält.

Enthält die erfindungsgemäße Zubereitung Ethylhexylsalicylat, so ist es erfindungsgemäß vorteilhaft, wenn diese Verbindung in einer Konzentration von 0,1 bis 5 Gewichts-%, bezogen auf das Gesamtgewicht der Zubereitung,

Enthält die erfindungsgemäße Zubereitung Homomenthylsalicylat, so ist es erfindungsgemäß vorteilhaft, wenn diese Verbindung in einer Konzentration von 0,1 bis 15 Gewichts-%, bezogen auf das Gesamtgewicht der Zubereitung,

Die erfindungsgemäße Zubereitung kann in unterschiedlichen Produktformen vorliegen, beispielsweise als Creme, Milch, Lotion oder Spray.

Eine erfindungsgemäß vorteilhafte Ausführungsform der vorliegenden Erfindung ist dabei dadurch gekennzeichnet, dass die Zubereitung sprühbar ist.

Als sprühbar wird eine Zubereitung erfindungsgemäß angesehen, wenn sie eine Viskosität von 300 bis 2000 mPas*s, aufweist. Dabei wird die erfindungsgemäße Viskosität wie folgt bestimmt: Messung bei 25°C im 150 ml Rollrandglas mittels Rheomat R123 von der Firma proRheo. Der Rheomat 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen das verwendete Messsystem wird die Viskosität berechnet. Messkörper Nr.3 (Artikelnr. 200 0193), geeignet für einen Viskositätsbereich bis 1.000 [mPas], Drehzahl Bereich 62,5 min.

Liegt die erfindungsgemäße Zubereitung in Form eines Sprays vor, so ist es erfindungsgemäß von Vorteil, wenn die Zubereitung kein Titandioxid oder Zinkoxid enthält.

Es ist erfindungsgemäß vorteilhaft, wenn die erfindungsgemäße Zubereitung Glycerin in einer Menge von 0,1 bis 15 Gewichts-%, bezogen auf das Gesamtgewicht der Zubereitung, enthält.

Es ist erfindungsgemäß bevorzugt, wenn die erfindungsgemäße Zubereitung Glycerin in einer Menge von 3 bis 10 Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung Vinylpyrrolidon/Triaconten-Copolymer in einer Menge von 0,01 bis 3 Gewichts-%, bezogen auf das Gesamtgewicht der Zubereitung, enthält.

Erfindungsgemäß bevorzugte Ausführungsformen der vorliegenden Erfindung sind dadurch gekennzeichnet, dass die Zubereitung Vinylpyrrolidon/Triaconten-Copolymer in einer Menge von 0,1 bis 2,0 Gewichts-%, bezogen auf das Gesamtgewicht der Zubereitung, enthält.

Erfindungsgemäß bevorzugt hat das erfindungsgemäße Vinylpyrrolidon/Triaconten-Copolymer einen Schmelzpunkt von 58-68 °C.

Erfindungsgemäß bevorzugt wird als Vinylpyrrolidon/Triaconten-Copolymer Antaron WP-660 der Firma Ashland Speciality Ingredients eingesetzt.

Es ist erfindungsgemäß von Vorteil, wenn die erfindungsgemäße Zubereitung in Form einer Emulsion vorliegt. Dabei sind O/W-Emulsionen (Öl-in-Wasser Emulsionen) die erfindungsgemäß bevorzugte Ausführungsform.

Liegt die erfindungsgemäße Zubereitung in Form einer Emulsion vor, so ist es vorteilhaft im Sinne der vorliegenden Erfindung, wenn die Zubereitung einen oder mehrere Emulgatoren gewählt aus der Gruppe der Verbindungen Glycerylstearatcitrat, Cetearylalkohol Natriumcetearylsulfat, Glycerylstearat, Cetearylsulfosuccinat Natriumstearoylglutamat, Polyglyceryl-3-methylglucosedistearat, Stearinsäure, Polyglyceryl-10 Stearat, Sucrose Polystearate, Kaliumcetylphosphat, enthält. Erfindungsgemäß bevorzugt ist es Einsatz der Emulgatoren Verbindungen Glycerylstearatcitrat, Cetearylsulfosuccinat, Sodium Cetearyl Sulfate, Natriumstearoylglutamat, Polyglyceryl-3-methylglucosedistearat, Polyglyceryl-10 Stearat, Polyglyceryl-6 Stearate sowie Kaliumcetylphosphat enthält.

Die erfindungsgemäß vorteilhafte Einsatzkonzentration (Gesamteinsatzkonzentration) für Emulgatoren beträgt dabei von 0,01 bis 4 Gewichts-%, bezogen auf das Gesamtgewicht der Zubereitung.

Erfindungsgemäß vorteilhafte Ausführungsformen der vorliegenden Erfindung sind dadurch gekennzeichnet, dass die Zubereitung Ethanol enthält.

Dabei ist eine Konzentration von 0,1 bis 20 Gewichts-% an Ethanol, bezogen auf das Gesamtgewicht der Zubereitung, erfindungsgemäß bevorzugt.

Es ist erfindungsgemäß vorteilhaft, wenn die erfindungsgemäße Zubereitung eine oder mehrere Verbindungen gewählt aus der Gruppe der Verbindungen Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

Dabei ist es erfindungsgemäß bevorzugt, wenn ein oder mehrere dieser Stoffe in Kombination mit Phenoxyethanol in der Zubereitung vorliegen. Besonders bevorzugte Kombinationen sind dabei die Kombinationen von Phenoxyethanol mit Propylenglycol, Butylenglycol, 1,2-Pentandiol, 1,2-Hexandiol und 1,2-Octandiol.

Es ist erfindungsgemäß vorteilhaft, wenn die Zubereitung einen oder mehrere Wirkstoffe gewählt aus der Gruppe der Verbindungen Magnolienextrakt, alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, ß-Alanin, Tocopherylacetat, Panthenol, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A, enthält.

Es ist erfindungsgemäß vorteilhaft, wenn die Emulsion frei ist von Parabenen, Isothiazolinonen und 3-lodpropargyl-N-butylcarbamat (IPBC).

Die Wasserphase der erfindungsgemäßen Emulsion kann übliche kosmetische Hilfsstoffe enthalten, ebenso wie die Ölphase, welche die üblichen Öle, Fette, Wachse, Lipide enthalten kann.

Allerdings ist es erfindungsgemäß von bevorzugt, wenn die Zubereitung C12-15 Alkylbenzoat, Cyclomethicon, Octyldodecanol, Capryl/Caprinsäure Triglyceride, Butylenglykol Dicaprylat/Dicaprat, Phenethylbenzoat, Cocoglyceride, Di-n-butyladipat und/oder Diisopropyladipat enthält.

Erfindungsgemäß besonders bevorzugt ist der Einsatz von Zubereitung Cyclomethicon, Octyldodecanol, Capryl/Caprinsäure Triglyceride, Butylenglykol Dicaprylat/Dicaprat, Phenethylbenzoat, Cocoglyceride, Di-n-butyladipat und/oder Diisopropyladipat

Dabei ist es erfindungsgemäß bevorzugt, wenn als Komplexbildner eine oder mehrere der Verbindungen aus der Gruppe
- Ethylendiamintetraessigsäure/ EDTA
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
und/oder deren Alkalisalze eingesetzt werden.

Erfindungsgemäß besonders bevorzugte Ausführungsformen der vorliegenden Erfindung sind dadurch gekennzeichnet, dass als Komplexbildner eine oder mehrere der Verbindungen aus der Gruppe
- Ethylendiamintetraessigsäure/ EDTA
- Diethylentriaminpenta(methylenphosphonsäure)/ DTPMP
- Ethylendiamintetra(methylenphosphonsäure/ EDTMP
- Aminotrimethylenphosphonsäure/ ATMP
- Iminodisuccinat
- Natriumpolyphosphat
- Tetranatriumpyrophosphat
- Bernsteinsäure
und/oder deren Alkalisalze eingesetzt werden.

Als erfindungsgemäß vorteilhafte Alkalisalze gelten dabei Natrium- und Kaliumsalze, wobei die Natriumsalze erfindungsgemäß bevorzugt werden.

Es ist vorteilhaft im Sinne der vorliegenden Erfindung, wenn die erfindungsgemäße Zubereitung einen oder mehrere Komplexbildner in einer Gesamtmenge von 0,1 bis 3 Gewichts-%, bezogen auf das Gesamtgewicht der Zubereitung, enthält.

Es ist bevorzugt im Sinne der vorliegenden Erfindung, wenn die erfindungsgemäße Zubereitung einen oder mehrere Komplexbildner in einer Gesamtmenge von 0,5 bis 1,5 Gewichts-%, bezogen auf das Gesamtgewicht der Zubereitung, enthält.

Darüber hinaus ist es vorteilhaft im Sinne der vorliegenden Erfindung, wenn die Zubereitung eine oder mehrere Verbindungen aus der Gruppe der Polysaccharide enthält.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Erfindungsgemäß vorteilhaft beträgt pH-Wert der erfindungsgemäßen Zubereitung 5-8.

Die erfindungsgemäße Zubereitung kann besonders bevorzugt als Tagespflegeprodukt oder Sonnenschutzmittel eingesetzt werden.

Erfindungsgemäß ist die Verwendung von Vinylpyrrolidon/Triaconten-Copolymer in Glycerinhaltigen kosmetischen Zubereitungen zur Verbesserung der Auswaschbarkeit von durch UV-A- und/oder Breitband UV-Filtern hervorgerufenen Textilverfleckungen.
Erfindungsgemäß ist nicht zuletzt die Verwendung von Vinylpyrrolidon/Triaconten-Copolymer in sprühbaren kosmetischen Zubereitungen, um die Tropfenbildung der zu versprühenden Zubereitung zu erhöhen und die Tropfengröße zu verkleinern, damit ein gleichmäßigfeinverteiltes Sprühbild entsteht.

### Vergleichsversuch 1

Mit dem folgenden Versuch konnte der erfindungsgemäße Effekt der verbesserten Auswaschbarkeit von durch UV-A- und/oder Breitband UV-Filtern hervorgerufenen Textilverfleckungen beispielhaft belegt werden:
Verfleckungsreduktion durch Zusatz von 0,5% bzw. 1,0% Vinylpyrrolidon/Triaconten-Copolymer zu einer o/w Basisemulsion ohne Vinylpyrrolidon/Triaconten-Copolymer (y = 0).

Als Beleg der verbesserten Auswaschbarkeit und verminderten Fleckenbildung der erfindungsgemäßen Zubereitungen wurden in vitro Untersuchungen durchgeführt, deren Ergebnisse in Tabelle 1 dargestellt sind.

Es wurden verschiedene Sonnenschutzemulsionen hinsichtlich der Bildung von gelben Flecken über einen in vitro Auftragungs-/ Waschzyklus untersucht. Es wurden dabei weiße vorgewaschene Baumwollmonitore (100% Baumwolle) verwendet. Dazu wurden je 50 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial- Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21 °C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel, 10 Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle 1: -db [%] Gelbwertreduktion im Vergleich zur Basis ohne Vinylpyrrolidon/Triaconten-Copolymer**

| **INCI** | **Beispiel [%]** | | |
|---|---|---|---|
| | Bsp. 1 | Bsp. 2 | Bsp. 3 |
| Triacontanyl PVP | | 0,50 | 1,00 |
| Cetearyl Alcohol | 1,00 | 1,00 | 1,00 |
| Glyceryl Stearate SE | 1,00 | 1,00 | 1,00 |
| Homosalate | 9,50 | 9,50 | 9,50 |
| Ethylhexyl Salicylate | 4,75 | 4,75 | 4,75 |
| Bis-Ethylhexyloxyphenol Methoxyphenyl Triazine | 3,50 | 3,50 | 3,50 |
| Butyl Methoxydibenzoylmethane | 4,75 | 4,75 | 4,75 |
| Myristyl Myristate | 1,00 | 1,00 | 1,00 |
| Phenoxyethanol | 0,50 | 0,50 | 0,50 |
| VP/Hexadecene Copolymer | 0,50 | | |
| Octocrylene | 9,50 | 9,50 | 9,50 |
| C18-38 Alkyl Hydroxystearoyl Stearate | 0,50 | 0,50 | 0,50 |
| Acrylates/C10-30 Alkyl Acrylate Crosspolymer | 0,10 | 0,10 | 0,10 |
| Xanthan Gum | 0,40 | 0,40 | 0,40 |
| Silica Dimethyl Silylate | 0,50 | 0,50 | 0,50 |
| Sodium Cetearyl Sulfate | 0,15 | 0,15 | 0,15 |
| Trisodium EDTA | 0,20 | 0,20 | 0,20 |
| Glycerin | 8,00 | 8,00 | 8,00 |
| Phenylbenzimidazole Sulfonic Acid | 1,00 | 1,00 | 1,00 |
| Alcohol Denat. | 4,00 | 4,00 | 4,00 |
| Methylparaben | 0,30 | 0,30 | 0,30 |
| Sodium Hydroxide | q.s. | q.s. | q.s. |
| Parfum | q.s. | q.s. | q.s. |
| Aqua | ad 100 | ad 100 | ad 100 |
| | | | |
| Gelbwert-Reduktion db [%] | - | -24 | -35 |

**Fazit:** Die Formulierungen mit Vinylpyrrolidon/Triaconten-Copolymer zeigen ein deutlich verringertes Verfleckungspotenzial im Vergleich zur Referenzformulierung.

### Vergleichsversuch 2

Mit dem folgenden Versuch konnte der erfindungsgemäße Effekt der vermehrten Tropfenbildung und der Verringerung der Tropfengröße in sprühbaren kosmetischen Zubereitungen durch den Einsatz von Vinylpyrrolidon/Triaconten-Copolymer belegt werden.

Als Beleg wurde, nach definierter Erzeugung eines Sprühbildes auf thermosensitivem Papier und Temperierung mittels Fön, dieses bildanalytisch ausgewertet.

### Methode zur Erstellung des Sprühbildes:

Die Messungen wurden unter folgenden Bedingungen durchgeführt:

| | |
|---|---|
| Gerät: | Druckluftsprühgestell |
| Druckregler: | 6 bar |
| Meßtemperatur.: | 25°C |
| Dämpfung: | Dämpfungsschraube geöffnet (Druckzylinder drückt schnell auf den Sprühkopf) |
| Sprühabstand: | 150 mm |
| Papier: | Thermopapier Art: 5702-1221 von e-pa |

Über eine mechanische Sprühvorrichtung wird das Produkt auf Thermopapier gesprüht. Anschließend wird das Thermopapier wird mit einem Fön erhitzt. Die nicht mit Produkt bedeckten Flächen erscheinen heller als der Rest vom Papier. So entstehen Kontrastbilder welche anschließend für die bildanalytische Auswertung herangezogen werden.

### Bildanalyse nach Kontrastunterschieden:

1. Definition der Region of Interest (ROI).
2. Auswertung des L-Werts (Helligkeit im L*a*b- Farbraum/ CIELab)
3. Binarisierung gemäß zuvor festgelegter Grenzwerte: schwarz (=0); weiß (=1)
4. Detektion der bedeckten Fläche (Area Count) und Berechnung der mittleren Größe (Area Average Size)
5. Berechnung der Absoluten Bedeckung (Absolute Coverage)
6. Berechnung der relativen Bedeckung (Relative Coverage)

| **Muster enthaltend** | **Bedeckte Fläche im Areal [%] (2)** | **Kreisgröße in der die Pixel bestimmt wurden** | **Anzahl der Einzel Pixel (4)** | **Durchschnittgröße der Bedeckung (5)** |
|---|---|---|---|---|
| VP/Hexadecene Copolymer (Vergleich) | 47,6 | 2793732 | 8203 | 193 |
| Vinylpyrrolidon/Triacont en-Copolymer | 49,68 | 2793732 | 8219 | 169 |

### Fazit:

Die absolut bedeckte Fläche des Sprühbildes (2) der erfindungsgemäßen Emulsion mit dem Rohstoff Vinylpyrrolidon/Triaconten-Copolymer ist in der gewählter ROI Size (1) größer.

Zusätzlich wird eine feinere Verteilung der erfindungsgemäßen Emulsion mit Triacontanyl PVP belegt durch eine höhere Anzahl der Einzelpixel (4) Pixel in der ROI size(1) bei gleichzeitig kleinerer Fläche (5) der Einzelpixel.

Durch den Einsatz von Vinylpyrrolidon/Triaconten-Copolymer findet somit eine feinere und gleichmäßigere Verteilung der Emulsion statt und es kann ein besserer Schutz vor schädlicher UV Strahlung gewährleistet werden.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen

| **INCI** | **Beispiel [%]** | | | | |
|---|---|---|---|---|---|
| | Bsp. 4 | Bsp. 5 | Bsp. 6 | Bsp. 4 | Bsp. 7 |
| C18-36 Acid Triglyceride | 1,00 | 1,00 | 0,50 | 1,00 | 2,00 |
| Butylene Glycol Dicaprylate/Dicaprate | 2,00 | 1,50 | 2,00 | 3,00 | 2,00 |
| Ceteareth-20 | 1,50 | 1,50 | 1,50 | 1,00 | 2,00 |
| Triacontanyl PVP | 0,80 | 1,00 | 1,00 | 1,50 | 1,00 |
| Parfum | q.s. | q.s. | q.s. | q.s. | q.s. |
| Glycerin | 5,00 | 5,00 | 5,00 | 5,00 | 5,00 |
| Sodium Hydroxide | q.s. | q.s. | q.s. | q.s. | q.s. |
| Phenoxyethanol | 0,50 | 0,50 | 0,50 | 0,50 | 0,50 |
| Ethylparaben | 0,20 | 0,20 | 0,20 | 0,20 | 0,20 |
| Methylparaben | 0,30 | 0,30 | 0,30 | 0,30 | 0,30 |
| Acrylates/C10-30 Alkyl Acrylate Crosspolymer | 0,30 | 0,40 | 0,60 | 0,40 | 0,10 |
| Alcohol Denat. | 4,00 | 4,00 | 2,00 | 6,00 | 4,00 |
| Trisodium EDTA | 0,20 | 0,20 | 0,20 | 0,20 | 0,20 |
| Homosalate | 9,00 | 8,00 | 7,00 | 9,00 | 5,00 |
| Octocrylene | 3,00 | 6,00 | 8,00 | 9,00 | |
| Ethylhexyl Salicylate | 4,80 | 4,80 | 4,80 | 5,00 | 5,00 |
| Bis-Ethylhexyloxyphenol Methoxyphenyl Triazine | 3,50 | 3,50 | 3,50 | 3,50 | 3,50 |
| Butyl Methoxydibenzoylmethane | 3,00 | 4,50 | 3,40 | 4,50 | 4,00 |
| Phenylbenzimidazole Sulfonic Acid | 1,50 | 2,00 | 2,50 | | 3,00 |
| Aqua | ad 100 | ad 100 | ad 100 | ad 100 | ad 100 |

| **INCI** | **Beispiel [%]** | | | | |
|---|---|---|---|---|---|
| | Bsp. 8 | Bsp. 9 | Bsp. 10 | Bsp. 11 | |
| Triacontanyl PVP | 1,00 | 1,00 | 1,00 | 1,00 | |
| C18-36 Acid Triglyceride | | | | 1,00 | |
| Dibutyl Adipate | 3,00 | | | | |
| C12-15 Alkyl Benzoate | 5,00 | | 2,00 | | |
| Butylene Glycol Dicaprylate/Dicaprate | 4,00 | | 2,00 | 2,00 | |
| C18-38 Alkyl Hydroxystearoyl Stearate | 1,00 | 0,50 | 1,00 | | |
| Myristyl Myristate | | 1,00 | | | |
| Glyceryl Stearate | 1,00 | 1,00 | 1,00 | | |
| Sodium Stearoyl Glutamate | 0,30 | | 0,30 | | |
| Glyceryl Stearate SE | | 1,00 | | | |
| Sodium Cetearyl Sulfate | | 0,15 | | | |
| Ceteareth-20 | | | | 1,50 | |
| Silica Dimethyl Silylate | 1,00 | 0,50 | 1,00 | | |
| Perfume | q.s. | q.s. | q.s. | q.s. | |
| Glycerin | 7,50 | 8,00 | 7,50 | 5,00 | |
| Sodium Hydroxide | q.s. | q.s. | q.s. | q.s. | |
| Phenoxyethanol | 0,50 | 0,50 | 0,50 | 0,50 | |
| Methylparaben | | 0,30 | | 0,30 | |
| Ethylparaben | | | | 0,20 | |
| Stearyl Alcohol | 1,00 | 1,00 | 1,00 | | |
| Acrylates/C10-30 Alkyl Acrylate Crosspolymer | 0,10 | 0,10 | 0,10 | 0,40 | |
| Xanthan Gum | 0,40 | 0,40 | 0,40 | 0,05 | |
| Cetearyl Alcohol | | 1,00 | | | |
| Alcohol Denat. | 3,00 | 4,00 | 3,00 | 4,00 | |
| Trisodium EDTA | 0,20 | 0,20 | 0,20 | 0,20 | |
| Ethylhexyl Salicylate | 4,75 | 4,75 | 4,75 | 4,75 | |
| Bis-Ethylhexyloxyphenol Methoxyphenyl Triazine | 4,00 | 3,50 | 4,00 | 3,50 | |
| Ethylhexyl Triazone | 3,00 | | 3,00 | | |
| Butyl Methoxydibenzoylmethane | 4,75 | 4,75 | 4,75 | 4,50 | |
| Phenylbenzimidazole Sulfonic Acid | 1,00 | 1,00 | 1,00 | 1,50 | |
| Homosalate | | 9,50 | 9,00 | 9,00 | |
| Octocrylene | | 9,50 | | 8,00 | |
| Aqua | ad 100 | ad 100 | ad 100 | ad 100 | |

## Patentansprüche

1. Kosmetische Zubereitung enthaltend
a) Glycerin und
b) Vinylpyrrolidon/Triaconten-Copolymer.

2. Kosmetische Zubereitung nach Anspruch 1, **dadurch gekennzeichnet, dass** die Zubereitung einen oder mehrere UV-Filter gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCl: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCl: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCl: Ethylhexyl Triazone); 2,4,6-Tribiphenyl-4-yl-1,3,5-triazin; Merocyanine; Titandioxid; Zinkoxid, enthält.

3. Kosmetische Zubereitung nach einem der vorhergehenden Ansprüche, **dadurch gekennzeichnet, dass** die Zubereitung sprühbar ist.

4. Kosmetische Zubereitung nach einem der vorhergehenden Ansprüche, **dadurch gekennzeichnet, dass** die Zubereitung Glycerin in einer Menge von 0,1 bis 15 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung Vinylpyrrolidon/Triaconten-Copolymer in einer Menge von 0,01 bis 3 Gewichts-%, bezogen auf das Gesamtgewicht der Zubereitung, enthält.

6. Kosmetische Zubereitung nach einem der vorhergehenden Ansprüche, **dadurch gekennzeichnet, dass** die Zubereitung in Form einer Emulsion vorliegt.

7. Kosmetische Zubereitung nach einem der vorhergehenden Ansprüche, **dadurch gekennzeichnet, dass** die Zubereitung einen oder mehrere Emulgatoren gewählt aus der Gruppe der Verbindungen Glycerylstearatcitrat, Cetearylalkohol Natriumcetearylsulfat + Glycerylstearat, Cetearylsulfosuccinat Natriumstearoylglutamat, Polyglyceryl-3-methylglucosedistearat, Stearinsäure, Polyglyceryl-10 Stearat, Kaliumcetylphosphat, enthält.

8. Kosmetische Zubereitung nach einem der vorhergehenden Ansprüche, **dadurch gekennzeichnet, dass** fei ist von 3-(4-Methylbenzyliden)-campher 2-Hydroxy-4-methoxybenzophenon (Oxybenzon) und 4-Methoxyzimtsäure(2-ethylhexyl)ester.

9. Kosmetische Zubereitung nach einem der vorhergehenden Ansprüche, **dadurch gekennzeichnet, dass** die Zubereitung 2-Phenylbenzimidazol-5-sulfonsäure und/oder deren Salze enthält.

10. Kosmetische Zubereitung nach einem der vorhergehenden Ansprüche, **dadurch gekennzeichnet, dass** die Zubereitung Ethylhexylsalicylat und/oder Homomenthylsalicylat enthält.

11. Kosmetische Zubereitung nach einem der vorhergehenden Ansprüche, **dadurch gekennzeichnet, dass** die Zubereitung einen oder mehrere UV-Filter gewählt aus der Gruppe der Verbindungen 4-(tert.-Butyl)-4'-methoxydibenzoylmethan, (2-[-4-(Diethylamino)-2-hydroxybenzoyl] Benzoesäurehexylester und 2,4-Bis-{[4-(2-ethyl-hexyl-oxy)-2-hydroxy]-phenyl}-6-(4-methoxyphenyl)-1,3,5-triazin (INCl Bis-Ethylhexyloxyphenol Methoxyphenyl Triazine), enthält.

12. Kosmetische Zubereitung nach einem der vorhergehenden Ansprüche, **dadurch gekennzeichnet, dass** die Zubereitung Ethanol enthält.

13. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Magnolienextrakt, alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, ß-Alanin, Tocopherylacetat, Panthenol, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A, enthält.

14. Kosmetische Zubereitung nach einem der vorhergehenden Ansprüche, **dadurch gekennzeichnet, dass** die Zubereitung C12-15 Alkylbenzoat, Cyclomethicon, Octyldodecanol, Capryl/Caprinsäure Triglyceride, Butylenglykol Dicaprylat/Dicaprat, Phenethylbenzoat, Cocoglyceride, Di-n-butyladipat und/oder Diisopropyladipat enthält.

15. Kosmetische Zubereitung nach einem der vorhergehenden Ansprüche, **dadurch gekennzeichnet, dass** die Zubereitung eine oder mehrere Verbindungen gewählt aus der Gruppe der Verbindungen Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

16. Kosmetische Zubereitung nach einem der vorhergehenden Ansprüche, **dadurch gekennzeichnet, dass** die Zubereitung einen oder mehrere Komplexbildner gewählt aus der Gruppe der Verbindungen
- Ethylendiamintetraessigsäure/ EDTA
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
und/oder deren Alkalisalze, und/oder ein oder mehrere Polysaccharide enthält.
